# EUROPÄISCHE PATENTSCHRIFT

(11) **EP 0 787 730 B1**
(45) Veröffentlichungstag und Bekanntmachung des Hinweises auf die Patenterteilung: **01.08.2001**
(21) Anmeldenummer: 97810030.3
(22) Anmeldetag: 22.01.1997
(51) Int. Cl.: C07D 487/04, C09B 57/00, C08K 5/3415

(54) **Polymerisierbare Diketopyrrolopyrrole und damit hergestellte Polymere**
Polymerisable diketopyrrolopyrroles and polymers prepared with same
Diketopyrrolopyrroles polyméisables et polymères préparés avec les-mêmes

(30) Priorität: 30.01.1996 CH 22896
(43) Veröffentlichungstag der Anmeldung: 06.08.1997
(73) Patentinhaber: Ciba Specialty Chemicals Holding Inc., 4057 Basel (CH)
(72) Erfinder: Eldin, Sameer Hosam, 1784 Courtepin (CH); Iqbal, Abul, 1732 Arconciel (CH); Hao, Zhimin, 1723 Marly (CH); Lamatsch, Bernd, 1723 Marly (CH)

(56) Entgegenhaltungen:
- EP-A- 0 563 901
- EP-A- 0 648 770
- EP-A- 0 656 403
- EP-A- 0 704 497
- CHEMICAL ABSTRACTS, vol. 122, no. 25, 19.Juni 1995 Columbus, Ohio, US; abstract no. 314058e, P. EDMAN ET. AL.: "Polarised Light Spectroscopy of Dihydropyrrolopyrroledione in Liquids and Liquid Crystals: Molecular Conformation and Influence by an Anisotropic Environment. " Seite 892; Spalte 2; XP002030112 & J. PHYS. CHEM., , Bd. 99, Nr. 21, Seiten 8504-9,

## Beschreibung

Die vorliegende Erfindung betrifft neue Diketopyrrolopyrrole mit zur Polyreaktion befähigten reaktiven Gruppen und damit hergestellte Polymere.

Die nunmehr auch in der Literatur, z.B. in Colour Index, als DPP-Pigmente bezeichneten und seit einigen Jahren bekannten und als wertvoll bewährten Diketopyrrolopyrrol-Pigmente sind u.a. in US-Patent 4 415 685 und US-Patent 4 579 949 beschrieben.

In EP-A 337 951 werden farbige Polymermikropartikel beschrieben, die durch Einpolymerisieren von zur Polymerisation befähigten reaktiven Gruppen enthaltenden Pigment-derivaten in verschiedenartigen Polymeren erhalten werden können. Es werden dabei Derivate der verschiedensten Pigmentklassen erwähnt, darunter auch DPP-Derivate, eines davon, ein an beiden Stickstoffatomen durch eine Ethylmethacrylatgruppe substituiertes 1,4-Diketo-3,6-diphenylpyrrolo[3,4-c]pyrrol, spezifisch.

Es hat sich jedoch erwiesen, dass die Einpolymerisierung dieser Verbindung nicht zufriedenstellend erfolgt.

Es ist nun gefunden worden, dass durch Einführung spezieller Reaktivgruppen, doch DPP-Chromophore erhalten werden können, die überraschend leicht zu oder mit Polymeren umgesetzt werden können, sei es direkt durch Homo- oder Copolymerisation oder auch durch Aufpfropfung auf bereits vorgebildeten Homo- oder Copolymeren.

Die vorliegende Erfindung betrifft demnach 1,4-Diketopyrrolopyrrole der Formel 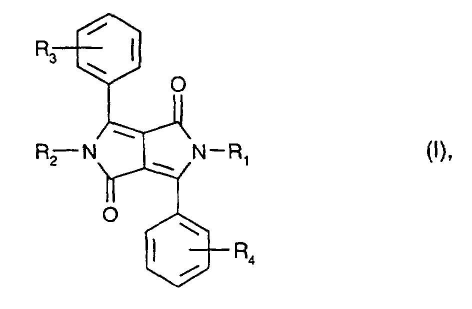 worin R₁ und R₂ unabhängig voneinander Wasserstoff, C₁₂-C₂₄-Alkyl, ein- oder mehrfach durch O oder S unterbrochenes C₆-C₂₄-Alkyl oder eine Gruppe der Formel 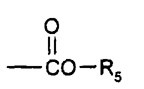 bedeuten, worin R₅ C₄-C₁₈-Alkyl oder C₅-C₁₀-Cycloalkyl ist,
R₃ OH, SH, NH₂, CHO, NCO, Hydroxyphenyl, -CH=CH₂, -CH=CH-COOR₆, 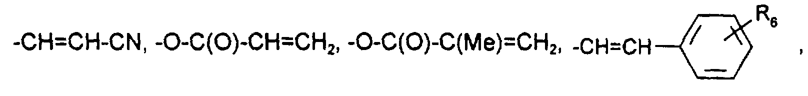 oder einen Rest der Formeln

-A-(CH₂)ₘ-CH=CH-(CH₂)ₙ-CH₃ (II),

oder

-A-(Y)ₚ-X-(Z)ᵣ-Q (III)

bedeutet, worin A -O- oder -NH- ist, m und n unabhängig voneinander eine ganze Zahl zwischen Null und 12 und p und r unabhängig voneinander Null oder 1 bedeuten,
X Methylen, ununterbrochenes oder ein- oder mehrfach durch -O- und/oder -S-, -NH-, Phenylen, -COO-, -CONH-, 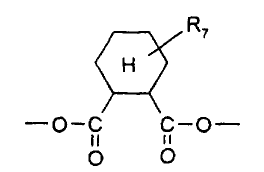 worin R₇ Wasserstoff oder Methyl ist, unterbrochenes C₂-C₁₈-Alkylen ist, 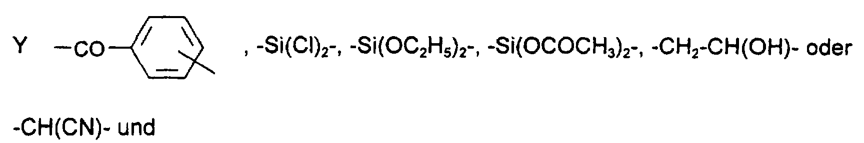 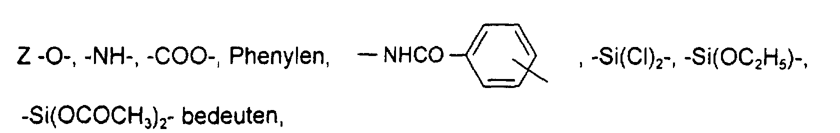 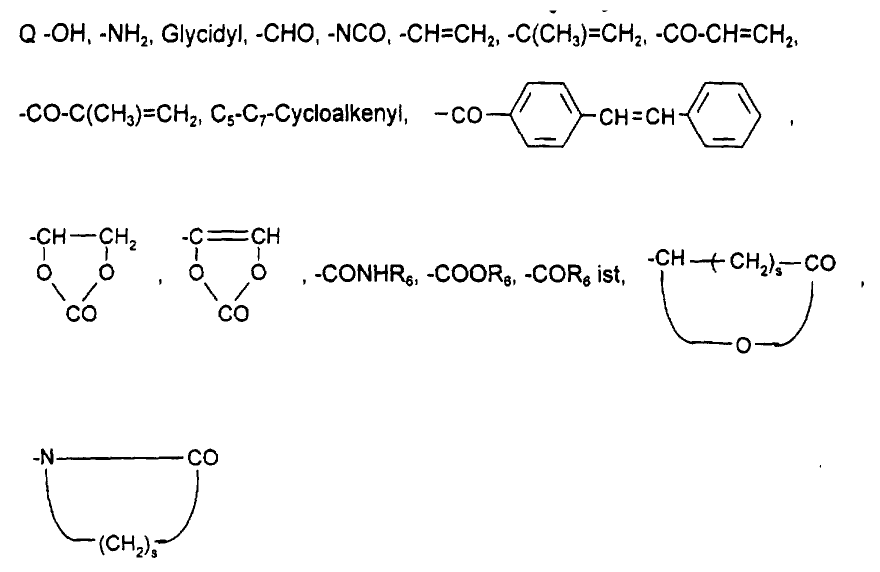 wobei s eine ganze Zahl von 1 bis 6 und R₆ Wasserstoff oder C₁-C₆Alkyl ist, bedeutet,

R₄, wenn R₁ und R₂ Wasserstoff bedeuten, ununterbrochenes oder ein- oder mehrfach durch O oder S unterbrochenes, direkt oder über O oder S am Benzolring gebundenes C₆-C₂₄-Alkyl ist und
wenn R₁ und/oder R₂ C₁₂-C₂₄-Alkyl, ein- oder mehrfach durch O oder S unterbrochenes C₆-C₂₄-Alkyl oder eine Gruppe 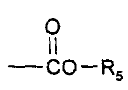 sind, Wasserstoff, Halogen, Methyl, Methoxy, CN, Phenyl oder gleich wie R₃ ist.

Unter zur Polyreaktion befähigten Unter zur Polymerisation befähigten reativen Gruppen versteht man z.B. zur Polymerisation befähigte Gruppen, wie z.B. Acrylatreste, oder zur Polykondensation befähigte Gruppen, wie z.B. Hydroxy- oder Säurechloridgruppen, oder auch zur Polyaddition befähigte Gruppen, wie z.B. Hydroxy- oder Isocyanatgruppen.
Bedeuten etwaige Substitutenten Halogen, dann handelt es sich z.B. um lod, Fluor, Chlor oder Brom, bevorzugt Brom oder Chlor, besonders bevorzugt Chlor;
C₁-C₄-Alkyl steht für Methyl, Ethyl, n-Propyl, iso-Propyl, n-Butyl, iso-Butyl, sek.-Butyl, tert.-Butyl;
C₁-C₆-Alkyl bedeutet beispielsweise Methyl, Ethyl, n-Propyl, iso-Propyl, n-Butyl, iso-Butyl, sek.-Butyl, tert.-Butyl, n-Amyl, tert.-Amyl, Hexyl;
C₄-C₂₄-Alkyl bedeutet beispielsweise n-Butyl, iso-Butyl, sek.-Butyl, tert.-Butyl, n-Amyl, tert.-Amyl, Hexyl, Heptyl, Octyl, 2-Ethylhexyl, Nonyl, Decyl, Dodecyl, Tetradecyl, Hexadecyl, Octadecyl, Eicosyl, Heneicosyl, Docosyl oder Tetracosyl;
C₆-C₁₈-Alkyl bedeutet beispielsweise Hexyl, Heptyl, Octyl, 2-Ethylhexyl, Nonyl, Decyl, Dodecyl, Tetradecyl, Hexadecyl, Octadecyl;
C₆-C₂₄-Alkyl bedeutet beispielsweise Hexyl, Heptyl, Octyl, 2-Ethylhexyl, Nonyl, Decyl, Dodecyl, Tetradecyl, Hexadecyl, Octadecyl, Eicosyl, Heneicosyl, Docosyl oder Tetracosyl;
C₁₂-C₁₈-Alkyl bedeutet beispielsweise Dodecyl, Tetradecyl, Hexadecyl, Octadecyl;

R₅ als C₄-C₁₈-Alkyl bedeutet geradkettiges oder, insbesondere bei kürzeren Ketten, verzweigtes Alkyl, wie z.B. tert.-Butyl, n-Pentyl, tert.-Amyl, n-Hexyl, 2,2-Dimethylbutyl, n-Octyl, 1,1,3,3-Tetramethylbutyl, 2-Ethylhexyl, Nonyl, Decyl, Dodecyl, Tetradecyl, Hexadecyl oder Octadecyl.

C₅-C₁₀-Cycloalkyl steht z.B. für Cyclopentyl, Cyclohexyl, Cycloheptyl, Trimethylcyclohexyl, insbesondere für Cyclohexyl.

C₅-C₇-Cycloalkenyl bedeutet beispielsweise mono- oder bicyclisches Cycloalkenyl, wie z.B. Cyclopentenyl, Cyclohexenyl oder Norbornenyl.

Einige Beispiele für den Rest -(Y)ₚ-X-(Z)ᵣ-Q in Formel lll sind -(CH₂)₆-OH, -(CH₂)₁₀-OH, -(CH₂)₁₁-OH, -(CH₂)₆-OCO-CH=CH₂, -(CH₂)₆-OCO-C(CH₃)=CH₂, -(CH₂)₁₀-OCO-CH=CH₂, -(CH₂)₁₀-OCO-C(CH₃)=CH₂, -(CH₂)₁₁-OCO-CH=CH₂, -(CH₂)₁₁-OCO-C(CH₃)=CH₂,
-(CH₂)₂-O-(CH₂)₂-O-(CH₂)₂-OH
-(CH₂)₂-O-(CH₂)₂-O-(CH₂)₂-O-CO-CH=CH₂
-(CH₂)₂-O-(CH₂)₂-O-(CH₂)₂-O-CO-C(CH₃)=CH₂ 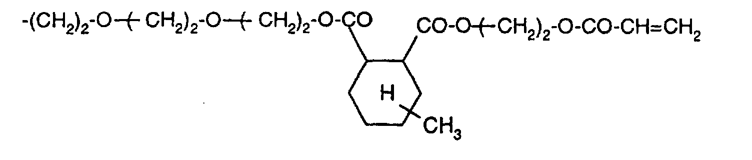 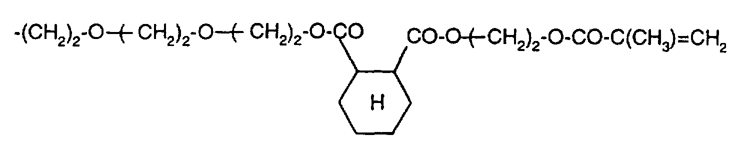 -(CH₂)₃-S-(CH₂)₂-OH
-(CH₂)₃-S-(CH₂)₆-OH
-(CH₂)₃-S-(CH₂)₂-COOH
-(CH₂)₃-S-(CH₂)₆-COOH
-(CH₂)₃-S-(CH₂)₂-NH₂
-(CH₂)₃-S-(CH₂)₆-NH₂
-(CH₂)₃-S-(CH₂)₂-O-(CH₂)₂-O-(CH₂)₂-OH
-(CH₂)₃-S-(CH₂)₂-O-(CH₂)₂-O-(CH₂)₂-NH₂ 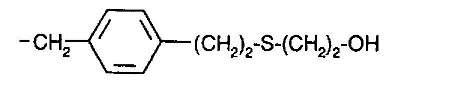 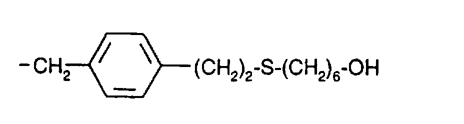 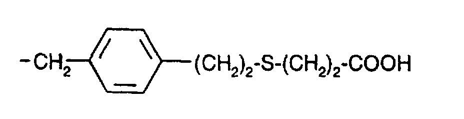 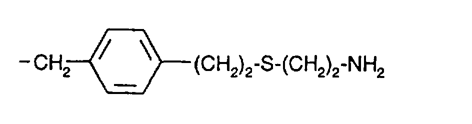 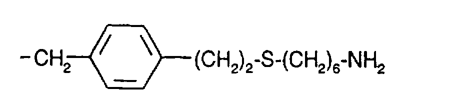 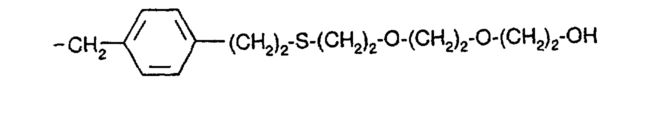 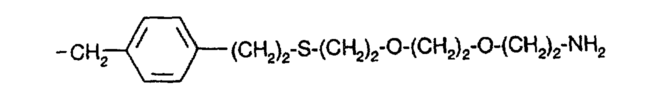 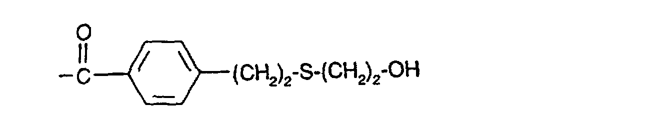 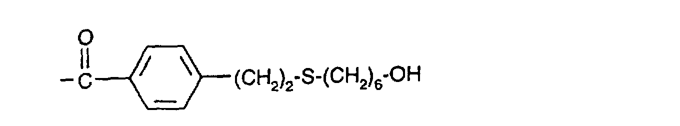 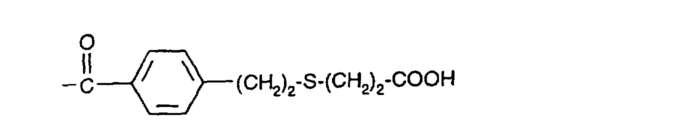 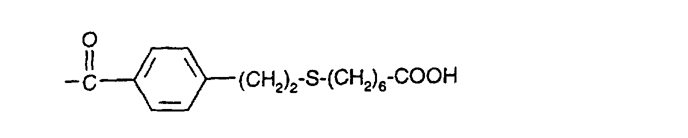 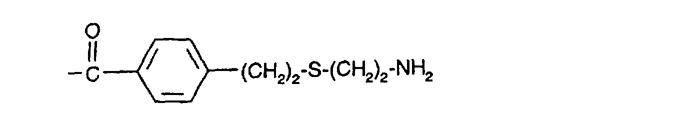 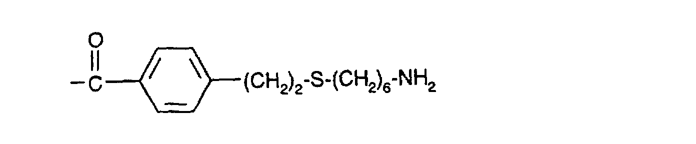 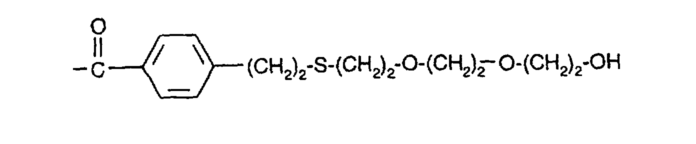 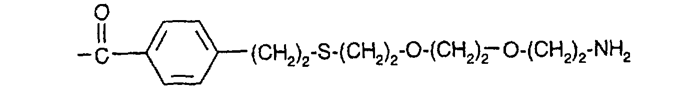 -(CH₂)₆-O-Si(Cl₂)-CH=CH₂
-(CH₂)₆-O-Si(OC₂H₅)₂-CH=CH₂
-(CH₂)₆-O-Si(O-COCH₃)₂-CH=CH₂
-Si(Cl)₂-(CH₂)₂-S-(CH₂)₂-OH
-Si(Cl)₂-(CH₂)₂-S-(CH₂)₆-OH
-Si(Cl)₂-(CH₂)₂-S-(CH₂)₂-COOH
-Si(Cl)₂-(CH₂)₂-S-(CH₂)₂-NH₂
-Si(Cl)₂-(CH₂)₂-S-(CH₂)₆-NH₂
-Si(Cl)₂-(CH₂)₂-S-(CH₂CH₂O)₂-CH₂CH₂OH
-Si(Cl)₂-(CH₂)₂-S-(CH₂CH₂O)₂-CH₂CH₂NH₂
-Si(OC₂H₅)₂-(CH₂)₂-S-(CH₂)₂-OH
-Si(OC₂H₅)₂-(CH₂)₂-S-(CH₂)₆-OH
-Si(OC₂H₅)₂-(CH₂)₂-S-(CH₂)₂-COOH
-Si(OC₂H₅)₂-(CH₂)₂-S-(CH₂)₆-COOH
-Si(OC₂H₅)₂-(CH₂)₂-S-(CH₂)₂-NH₂
-Si(OC₂H₅)₂-(CH₂)₂-S-(CH₂)₆-NH₂
-Si(OC₂H₅)₂-(CH₂)₂-S-(CH₂CH₂O)₂-CH₂CH₂OH
-Si(OC₂H₅)₂-(CH₂)₂-S-(CH₂CH₂O)₂-CH₂CH₂NH₂
-Si(OCOCH₃)₂-(CH₂)₂-S-(CH₂)₂-OH
-Si(OCOCH₃)₂-(CH₂)₂-S-(CH₂)₆-OH
-Si(OCOCH₃)₂-(CH₂)₂-S-(CH₂)₂-COOH
-Si(OCOCH₃)₂-(CH₂)₂-S-(CH₂)₆-COOH
-Si(OCOCH₃)₂-(CH₂)₂-S-(CH₂)₂-NH₂
-Si(OCOCH₃)₂-(CH₂)₂-S-(CH₂)₆-NH₂
-Si(OCOCH₃)₂-(CH₂)₂-S-(CH₂CH₂O)₂-CH₂CH₂OH
-Si(OCOCH₃)₂-(CH₂)₂-S-(CH₂CH₂O)₂-CH₂CH₂NH₂
-CH₂CH(OH)-CH₂-S-(CH₂)₆-COOH
-CH₂CH(OH)-CH₂-S-(CH₂)₆-OH
-CH₂CH(OH)-CH₂-S-(CH₂CH₂O)₂-CH₂CH₂OH
-CH₂CH(OH)-CH₂-S-(CH₂CH₂O)₂-CH₂CH₂COOH
-CH₂CH(OH)-CH₂-NH-(CH₂)₆-COOH
-CH₂CH(OH)-CH₂-NH-(CH₂)₆-OH
-CH₂CH(OH)-CH₂-NH-(CH₂CH₂O)₂-CH₂CH₂OH
-CH₂-CONH-(CH₂)₆-OH
-CH₂-CONH-(CH₂)₆-COOH
-CH₂-CONH-(CH₂CH₂O)₂-CH₂CH₂OH 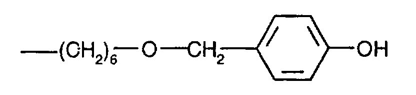 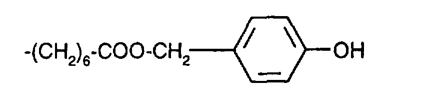 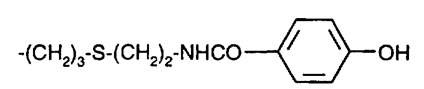 -CH(CN)-(CH₂)₆-OH
-CH(CN)-(CH₂CH₂O)₂-CH₂CH₂OH
-(CH₂)₆-O-CH₂CH₂O-CH=CH₂
-(CH₂)₆-O-CH₂-CH=CH₂
-(CH₂)₆-O-CH=CH₂
-(CH₂)₆-O-CH₂-CO-CH=CH₂
-(CH₂)₆-O-CO-CH=CH₂
-(CH₂)₆-O-(CH₂)₂-NHCO-CH=CH₂
-(CH₂)₆-O-CH₂-COO-CH=CH₂ 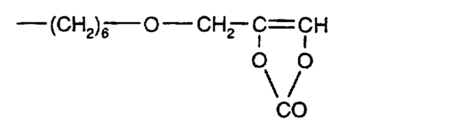 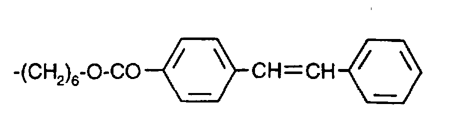 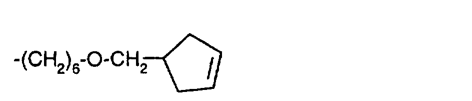 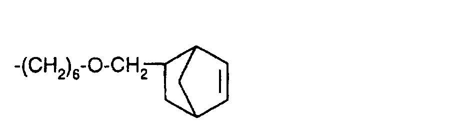 -(CH₂)₆-O-CH₂-CHO
-(CH₂)₆-O-CH₂-NCO 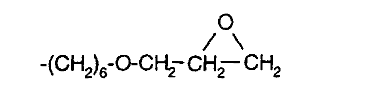 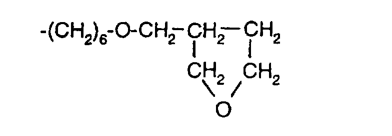 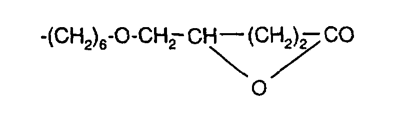 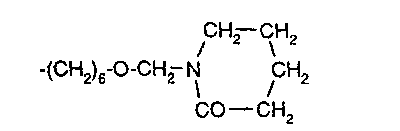 -(CH₂)₃-S-(CH₂)₂-CO-O-CO-CH₃.

Von besonderem Interesse sind die erfindungsgemässen Diketopyrrolopyrrole der Formel I, worin R₃ und R₄ in Parastellung stehen.

Bevorzugt werden erfindungsgemässe Diketopyrrolopyrrole der Formel l, worin R₁ und R₂ Wasserstoff sind und R₄ direkt oder über O am Benzolring gebundenes C₆-C₁₈-Alkyl oder eine Gruppe -O(CH₂CH₂O)ₓCH₂CH₃ bedeutet, worin x 1, 2 oder 3 ist.

Ebenfalls bevorzugt werden erfindungsgemässe Diketopyrrolopyrrole der Formel l, worin mindestens einer der Reste R₁ und R₂ C₁₂-C₁₈-Alkyl, eine Gruppe (CH₂CH₂O)ₓCH₂CH₃, worin x die oben angegebene Bedeutung hat oder eine Gruppe der Formel 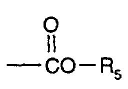 bedeutet,
- R₃: die oben angegebene Bedeutung hat und
- R₄: Wasserstoff oder die gleiche Bedeutung wie R₃ hat.
- R₃: bedeutet besonders vorzugsweise OH, NH₂ oder einen Rest der Formel

-X-(O)ᵣ-Q (IV),

worin
- x: ununterbrochenes oder 1, 2 oder 3 Mal durch O und/oder einmal durch

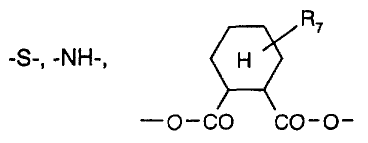

unterbrochenes C₄-C₁₂-Alkylen ist,
r und R₇ die oben angegebene Bedeutung haben, und
Q -OH; -CH=CH₂, -C(CH₃)=CH₂, -CO-CH=CH₂ oder -CO-C(CH₃)=CH₂ bedeutet.
X bedeutet vorzugsweise -(CH₂)_{q}-, wobei q eine ganze Zahl zwischen 6 und 12 wie 6, 7, 8, 9, 10, 11 oder 12 sein kann,
   -(CH₂CH₂O)₂-CH₂CH₂- oder

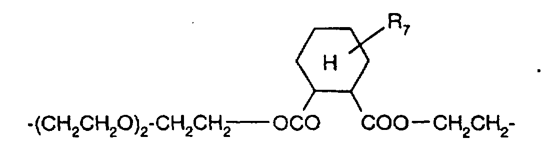

Die erfindungsgemäßen Diketopyrrolopyrrole stellt man bevorzugt durch Umsetzung eines Bernsteinsäurediesters mit einem Nitril her, indem man (Schritt a) einen unsymmetrischen oder symmetrischen Bernsteinsäure-dialkyl- oder -diaryl-ester, oder einen Bernsteinsäure-monoalkyl-monoaryl-ester oder Bernsteinsäuredicyclohexylester mit einem Nitril der Formel 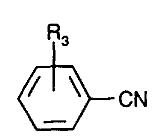 oder mit einer Mischung, bevorzugt einer äquimolaren Mischung, der Nitrile der Formeln 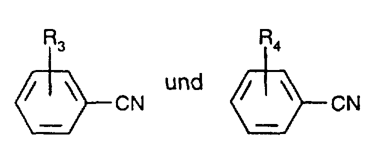 wobei R₃ und R₄ die gleiche Bedeutung wie oben haben sowie zusätzlich übliche Schutzgruppen für beispielsweise die -OH-Gruppe oder zur Erzeugung einer -CHO-Gruppe bedeuten können wie C₁-C₄-Alkoxy, insbesondere Methoxy, bevorzugt in para-Position, und die 1,3-Dioxan-2-yl-Gruppe, bevorzugt in para-Position, im gewünschten Molverhältnis in einem organischen Lösungsmittel in Gegenwart einer starken Base wie ein Alkalimetall, insbesondere Natrium, Alkalimetallamid wie Natriumamid, Alkalimetallhydrid wie Natriumhydrid, Alkalimetallalkoholat, insbesondere mit einem C₁-C₅-Alkanol, wie NaOMe, NaO-tert.-amyl, umsetzt, und anschließend das Reaktionsprodukt hydrolysiert und gewünschtenfalls das gewünschte Produkt isoliert.

Gewünschtenfalls kann man in einem Schritt (b) die so erhaltenen Diketopyrrolopyrrole der Formel I, in denen R₁ und R₂ für Wasserstoff stehen, mit (b1) einem Dicarbonat der Formel XI, 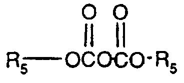 im gewünschten Molverhältnis umsetzen, oder (b2) im gewünschten Molverhältnis mit einer Halogenverbindung der Formel Xll, R₁-Hal, worin R₁ C₁₂-C₂₄-Alkyl, ein- oder mehrfach durch O oder S unterbrochenes C₆-C₂₄-Alkyl ist, umsetzen, oder (b3) im gewünschten Molverhältnis mit einer Halogenverbindung wie Hal-(CH₂)ₘ-CH=CH-(CH₂)ₙ-CH₃, oder Hal-(Y)ₚ-X-(Z)ᵣ-Q, umsetzen, worin m, n, p, r, Y, X, Q und Z die gleiche Bedeutung wie oben haben und Hal für Fluor, Chlor, Brom oder lod, insbesondere für Chlor oder Brom steht.

Des weiteren kann man in einem dritten Schritt (c) direkt vor oder nach Schritt (b) gewünschtenfalls Diketopyrrolopyrrole der Formel I, in denen R₃ und/oder R₄ Schutzgruppen sind, bevorzugt die Methoxygruppe oder die 1,3-Dioxan-2-yl-Gruppe, in üblicher Weise, beispielsweise durch Hydrolyse, zum entsprechenden gewünschten Zielmolekül umsetzen. So kann man beispielsweise die Methoxygruppe in die -OH-Gruppe und die 1,3-Dioxan-2-yl-Gruppe in eine -CHO-Gruppe nach bekannten Methoden umwandeln.

Die Wahl der Reaktionsparameter kann man beispielsweise analog zu dem in der US 4,579,949 beschriebenen Verfahren vornehmen, so daß sich nähere Angaben hierzu erübrigen.

Des weiteren kann man in einer bevorzugten Ausführungsform die erfindungsgemässen Diketopyrrolopyrrole der Formel I, worin R₁ und R₂ Wasserstoff bedeuten, z.B. durch Umsetzung eines Pyrrolinons der Formel 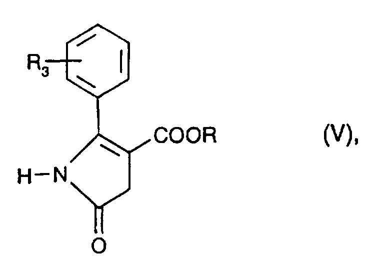 worin R z.B C₁-C₄-Alkyl ist, mit einem Nitril der Formel 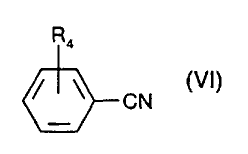 wobei R₃ und R₄ die oben angegebene Bedeutung haben, in Analogie zu dem im US-Patent 4 778 899 beschriebenen Verfahren, herstellen.

Pyrrolinone der Formel V erhält man üblicherweise nach an sich bekannten Methoden, z.B. durch Cyclisierung einer Verbindung der Formel 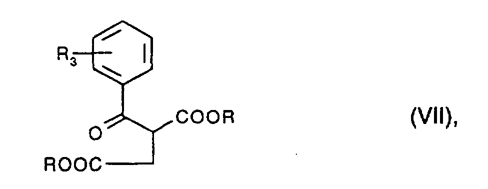 worin R₃ und R die oben angegebene Bedeutung haben, mit einem Ammoniumsalz, wie z.B. im US-Patent 4 778 899 beschrieben.

Die Verbindungen der Formel Vll sowie die Nitrile der Formel Vl sind bekannt und/oder können nach an sich bekannten Methoden hergestellt werden.

Nitrile der Formel Vl worin R₄ über O oder S gebundenes, ununterbrochenes oder durch O oder S unterbrochenes C₄-C₂₄-Alkyl bedeutet, können z.B. aus Hydroxybenzonitril durch Umsetzung mit einer Halogenverbindung der Formel R₄-Hal nach bekannten Methoden erhalten werden. In analoger Weise können Verbindungen der Formel Vll, worin R₃ eine Gruppe

-A-(CH₂)ₘ-CH=CH-(CH₂)ₙ-CH₃ (II)

oder

-A-(Y)ₚ-X-(Z)ᵣ-Q (III)

bedeutet, durch Umsetzung eines Diesters der Formel 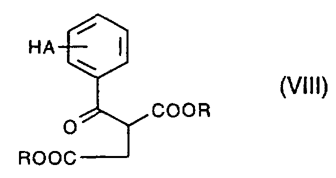 worin A und R die oben angegebene Bedeutung haben, mit einer Halogenverbindung der Formel

Hal-(CH₂)ₘ-CH=CH-(CH₂)ₙ-CH₃ (IX)

oder

Hal-(Y)ₚ-X-(Z)ᵣ-Q (X)

erhalten werden, wobei es sich bei den Verbindungen der Formeln Vlll, IX und X um bekannte oder für den Fachmann jedenfalls leicht zugängliche Substanzen handelt. Hal bedeutet Halogen wie Fluor, Chlor, Brom oder lod, bevorzugt Chlor oder Brom, besonders bevorzugt Chlor.

Erfindungsgemässe Diketopyrrolopyrrole der Formel l, worin R₁ und R₂ Wasserstoff und R₃ gegebenenfalls auch R₄ -CH=CH-COOR₆, -CH=CH-CN oder 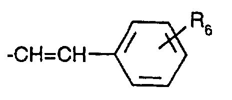 bedeuten, kann man in einer weiteren bevorzugten Ausführungsform ebenfalls nach bekannten Methoden, z.B. nach R.F. Heck, Organic Reactions, 27, 345 (1982) aus Diketopyrrolopyrrolen der Formel l, worin R₁ und R₂ Wasserstoff und R₃ bzw. R₄ Halogen bedeuten, herstellen.

Erfindungsgemässe Diketopyrrolopyrrole der Formel l, worin R₁ und/oder R₂ eine Gruppe 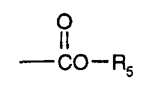 bedeuten, können beispielsweise in einer weiteren bevorzugten Ausführungsform nach an sich bekannten Verfahren, wie z.B. in EP-A 0 648 770 beschrieben, durch Umsetzung entsprechender Diketopyrrolopyrrole der Formel l, worin R₁ und R₂ Wasserstoff bedeuten, im gewünschten Molverhältnis mit einem Dicarbonat der Formel 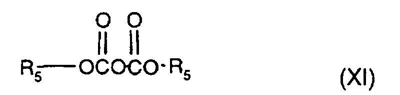 erhalten werden.

Dicarbonate der Formel Xl sind bekannt und/oder nach bekannten Methoden zugängliche Verbindungen.

Erfindungsgemässe Diketopyrrolopyrrole der Formel l, worin R₁ und/oder R₂ C₁₂-C₂₄-Alkyl, ein- oder mehrfach durch O oder S unterbrochenes C₆-C₂₄-Alkyl bedeuten, können in einer weiteren bevorzugten Ausführungsform nach an sich bekannten Methoden durch Umsetzung entsprechender Diketopyrrolopyrrole der Formel l, worin R₁ und R₂ Wasserstoff bedeuten, im gewünschten Molverhältnis mit einer Halogenverbindung der Formel

R₁-Hal (XII),

worin R₁ C₁₂-C₂₄-Alkyl, ein- oder mehrfach durch O oder S unterbrochenes C₆-C₂₄-Alkyl ist, erhalten werden.

Wird beispielsweise 1 Moläquivalent der Verbindung der Formel Xl oder Xll eingesetzt, so erhält man üblicherweise ein DPP der Formel l, worin R₂ Wasserstoff bedeutet, werden beispielsweise 2 Moläquivalente eingesetzt, dann erhält man im allgemeinen ein DPP-Derivat, in dem die Reste R₁ und R₂ gleich sind.

Bedeutet R₃ in erfindungsgemässen Diketopyrrolopyrrolen dieses Typs eine Gruppe der Formeln II oder lll, so kann diese Gruppierung durch Umsetzung mit Halogenverbindungen der Formeln IX bzw. X, in Anlehnung an die oben im Zusammenhang mit der Herstellung der Verbindungen der Formel Vll beschriebenen Methode, vor oder bevorzugt nach der Umsetzung mit dem Dicarbonat, eingeführt werden.

In analoger Weise können durch Umsetzung von Diketopyrrolopyrrolen der Formel l, worin R₁ und R₂ Wasserstoff bedeuten, im gewünschten Molverhältnis mit Halogenverbindungen der Formeln IX und X, auch Diketopyrrolopyrrole der Formel 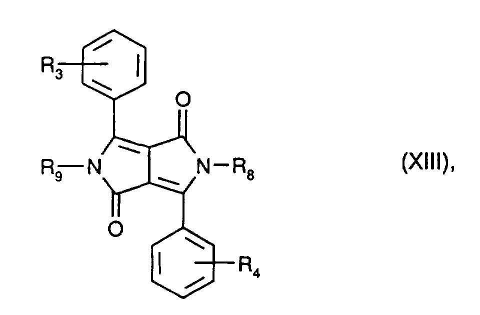 worin R₈ eine Gruppe

-(CH₂)ₘ-CH=CH-(CH₂)ₙ-CH₃

oder

-(Y)ₚ-X-(Z)ᵣ-Q

bedeutet und R₉ Wasserstoff oder R₈ ist, und R₃, R₄, X, Y, Z, Q, m, n, p und r die oben angegebene Bedeutung haben, hergestellt werden.

Die Diketopyrrolopyrrole der Formel Xlll, die insbesondere zur Herstellung mehrfach vernetzter Polymere geeignet sind, bilden einen weiteren Gegenstand der vorliegenden Anmeldung.

Die erfindungsgemäßen DPP-Verbindungen kann man dank ihrer reaktiven Gruppen sehr gut zur Herstellung oder Modifizierung von Polymeren verwenden. Die so hergestellten oder modifizierten farbigen Polymere weisen unerwartet interessante Farbeffekte und, je nach der Menge der eingesetzten DPP-Verbindung, verschiedenartigste Nuancen auf, die von jenen der entsprechenden DPP-Pigmente, die keine zu Polyreaktionen befähigten Gruppen besitzen, völlig abweichen können.

Des weiteren betrifft die vorliegende Erfindung ein Verfahren zur Herstellung oder Modifizierung von Polymeren durch Polyreaktion oder polymeranaloge Reaktion, indem man ein Diketopyrrolopyrrol der Formel l, gewünschtenfalls in Gegenwart eines üblichen, z.B. mindestens eine Kohlenstoff-Kohlenstoff-Doppelbindung tragenden Comonomeren oder eines polyreaktionsfähige Gruppen tragenden Polymeren, polymerisiert.

In einer bevorzugten Ausführungsform kann man gefärbte (Co-)Polymere herstellen, indem man eine Mischung aus erfindungsgemäßen DPP-Monomeren und weiteren, üblichen und geeigneten Monomeren in flüssiger Phase wie in Schmelze, Lösung, Suspension und Emulsion zur Polyreaktion bringt.

Die Herstellung dieser neuen DPP-Polymeren erfolgt üblicherweise nach allgemein bekannten Methoden, beispielsweise entweder durch eine Polyreaktion, d.h. durch Polymerisation (thermisch oder photochemisch), Polykondensation oder Polyaddition, oder durch eine polymeranaloge Reaktion, d.h. durch Umsetzung der erfindungsgemässen geeignete reaktive Gruppen enthaltenden DPP-Verbindungen mit bereits vorliegenden Polymeren, die selbst reaktionsfähige Gruppen aufweisen (Pfropfung).

Nach bisherigen Beobachtungen kann man mit den erfindungsgemässen DPP-Verbindungen (DPP-Monomeren) alle bekannten Polyreaktionen durchführen. So können z.B. aus DPP-Monomeren, deren reaktive Gruppen C=C-Bindungen aufweisen, Vinyl-, Allyl-, Vinylester-, Vinylamid-, Vinylacetat- oder Vinylketon-Polymere, aus monofunktionellen DPP-Monomeren, deren reaktive Gruppen Heteroatome enthalten, Polyaldehyde, Polyisocyanate, Polyepoxide, Polyether, Polyacetone oder Polylactame, aus bifunktionellen DPP-Monomeren, deren reaktive Gruppen Heteroatome enthalten, via Polykondensation Polyester, Polyamide, Polyimide oder Polycarbonate und via Polyaddition, Polyepoxide, Polyurethane oder Polyimide hergestellt werden, wobei es sich bei der Polymerisation um radikalische, kationische oder anionische Polymerisation, Coordinationspolymerisation oder Gruppentransferpolymerisation handeln kann.
Beispiele für die Herstellung von DPP-Polymeren ausgehend von erfindungsgemässen DPP-Monomeren sind:
Polymerisation: DPP-Polyacrylate durch radikalische thermische Polymerisation von DPP Acrylaten; DPP-Polyacrylate durch radikalische Photopolymerisation von DPP-Acrylaten.
Polykondensation: DPP-Polyester aus DPP-Diolen und Disäurechloriden; DPP-Polycarbonate aus DPP-Diolen und Phosgen.
Polyaddition: DPP-Polyurethane aus DPP-Diolen und Diisocyanaten; DPP-Polyepoxide aus DPP-Epoxiden und Aminen
Polymeranaloge Reaktion: Umsetzung eines DPP-Alkohols mit einem aus Styrol und Maleinsäureanhydrid hergestellten und demnach Anhydridgruppen aufweisenden Polymer zu einem DPP-Mono- oder -diestergruppen enthaltenden Polymer.

Gegebenenfalls enthalten die neuen DPP-Polymeren auch Additive, wie Lichtschutzmittel, Antioxidantien und UV-Absorber, die während oder nach der eigentlichen Polyreaktion zugegeben werden können, z.B. auch bei der Verarbeitung der Polymeren (Extrusion). Diese Additive können auch selbst polyreaktionsfähige reaktive Gruppen aufweisen und in diesem Fall zusammen mit den DPP-Monomeren copolymerisiert werden.

Die erfindungsgemäß hergestellten DPP-Polymere, worunter im folgenden auch Copolymere, hergestellt aus erfindungsgemäßen DPP-Monomeren und anderen, üblichen Monomeren, zu verstehen sind, eignen sich vorteilhaft für viele Zwecke wie zur Einfärbung von hochmolekularen organischen Materialien wie Biopolymeren, Kunststoffen, inklusive Fasern, Gläsern, keramischen Produkten, für Zubereitungen in der dekorativen Kosmetik, zur Herstellung von Tinten, Druckfarben, Lacken, insbesondere Automobillacken und Photolacken, photo- und elektroleitenden Polymeren, fluoreszierenden Aufhellern, Photozellen-Aggregaten, gefärbten Photoresists und Dispersionsfarben, des weiteren kann man die erfindungsgemäßen Diketopyrrolopyrrole im biomedizinischen Anwendungsbereich einsetzen wie zur Herstellung von Diagnostika sowie auf den Gebieten Impact- und Non-Impact-Printing und Photo/Repro allgemein.

Beispiele geeigneter hochmolekularer organischer Materialien, die mit den erfindungsgemäßen DPP-Polymeren gefärbt werden können, sind Vinylpolymere wie Polystyrol, Poly-α-methylstyrol, Poly-p-methylstyrol, Poly-p-hydroxystyrol, Poly-p-hydroxyphenylstyrol, Polymethylmethacrylat und Polyacrylamid sowie die entsprechenden Methacrylverbindungen, Polymethylmaleat, Polyacrylnitril, Polymethacrylnitril, Polyvinylchlorid, Polyvinyl-fluorid, Polyvinylidenchlorid, Polyvinylidenfluorid, Polyvinylacetat, Polymethylvinylether und Polybutylvinylether; von Maleinimid und/oder Maleinanhydrid abgeleitete Polymere wie Copolymere von Maleinanhydrid mit Styrol; Polyvinylpyrrolidon; ABS; ASA; Polyamide; Polyimide; Polyamidimide; Polysulfone; Polyethersulfone; Polyphenylenoxide; Polyurethane; Polyharnstoffe; Polycarbonate; Polyarylene; Polyarylensulfide; Polyepoxide; Polyolefine wie Polyethylen und Polypropylen; Polyalkadiene; Biopolymere und deren Derivate wie Cellulose, Celluloseether und -ester wie Ethylcellulose, Nitrocellulose, Celluloseacetat und Cellulosebutyrat, Stärke, Chitin, Chitosan, Gelatine, Zein; natürliche Harze; Kunstharze wie Alkydharze, Acrylharze, Phenolharze, Epoxidharze, Aminoformaldehydharze wie Harnstoff- und Melamin-Formaldehydharze; Gummi; Casein; Silikon und Silikonharze; Kautschuk, Chlorkautschuk; des weiteren Polymere, die beispielsweise als Bindemittel in Lacken verwendet werden wie Novolacke abgeleitet von C₁-C₆-Aldehyden wie Formaldehyd und Acetaldehyd und einem zweikernigen oder einkernigen, vorzugsweise einkernigen Phenol, das gewünschtenfalls mit einer oder zwei C₁-C₉-Alkylgruppen, einem oder zwei Halogenatomen oder einem Phenylring substituiert ist wie o-, m- oder p-Kresol, Xylol, p-tert.-Butylphenol, o-, m- oder p-Nonylphenol, p-Chlorphenol oder p-Phenylphenol, oder einer Verbindung mit mehr als einer phenolischen Gruppe wie Resorcin, Bis-(4-hydroxyphenyl)methan oder 2,2-Bis-(4-hydroxyphenyl)propan; sowie geeignete Mischungen der genannten Materialien.

Besonders bevorzugte hochmolekulare organische Materialien, insbesondere zur Herstellung eines Lackes, einer Druckfarbe oder Tinte, sind beispielsweise Celluloseether und -ester, wie Ethylcellulose, Nitrocellulose, Celluloseacetat und Cellulosebutyrat, natürliche Harze oder Kunstharze (Polymerisations- oder Kondensationsharze) wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, ASA, Polyphenylenoxide, Gummi, Casein, Silikon und Silikonharze sowie deren mögliche Mischungen untereinander.

Man kann auch hochmolekulare organische Materialien in gelöster Form als Filmbildner einsetzen wie Leinölfimis, Nitrocellulose, Alkydharze, Phenolharze, Melamin- und Harnstoff/Formaldehydharze sowie Acrylharze.

Die genannten hochmolekularen organischen Verbindungen können einzeln oder in Gemischen z.B. als Granulat, plastische Massen, Schmelzen oder in Form von Lösungen, insbesondere zur Herstellung von Spinnlösungen, Lacken, Anstrichstoffen, Tinten oder Druckfarben, vorliegen.

In einer besonders bevorzugten Ausführungsform verwendet man die erfindungsgemäßen DPP-Polymere zum Massefärben von Polyvinylchlorid, Polyamiden und insbesondere Polyolefinen wie Polyethylen und Polypropylen sowie zur Herstellung von Lacken, inklusive Pulverlacken, Tinten, Druckfarben und Anstrichfarben.

Als Beispiele für bevorzugte Bindemittel für Lacksysteme seien Alkyd/Melaminharzlacke, Acryl/Melaminharzlacke, Celluloseacetat/Cellulosebutyratlacke und Zweikomponentenlacke auf Basis mit Polyisocyanat vernetzbarer Acrylharze genannt. Nach bisherigen Beobachtungen kann man die erfindungsgemäßen DPP-Polymere in jeder gewünschten Menge in Abhängigkeit vom Verwendungszweck dem zu färbenden Material zugeben. Beispielsweise kann man bei hochmolekularen organischen Materialien die erfindungsgemäßen zusammengesetzten Pigmente in einer Menge im Bereich von 0,01 bis 40, bevorzugt von 0,1 bis 20 Gew.-%, bezogen auf das Gesamtgewicht des gefärbten hochmolekularen organischen Materials, einsetzen.

Die Einfärbung der hochmolekularen organischen Materialien mit den erfindungsgemäßen DPP-Polymeren erfolgt in der Regel dergestalt, daß man die erfindungsgemäßen DPP-Polymere, gewünschtenfalls in Form von Masterbatches, den hochmolekularen organischen Materialien unter Verwendung üblicher hierzu geeigneter Vorrichtungen wie Extruder, Walzwerke, Misch- oder Mahlapparaturen zumischt. Das so behandelte Material wird im allgemeinen hierauf nach an sich bekannten Verfahren wie Kalandrieren, Pressen, Strangpressen, Streichen, Gießen, Extrudieren oder Spritzgießen in die gewünschte endgültige Form gebracht.

In einer bevorzugten Ausführungsform kann man die erfindungsgemäßen DPP-Monomeren zusammen mit anderen Monomeren, insbesondere solchen, die üblicherweise zur Herstellung der bereits weiter oben genannten Polymere eingesetzt werden, in einem Extruder zur Polyreaktion bringen (reactive extrusion, analog zu beispielsweise dem in der EP-A 337 951 beschriebenen Verfahren). So hergestellte Copolymere weisen üblicherweise die gleiche Anwendungsbreite auf wie die bislang genannten Blends aus erfindungsgemäßen DPP-Polymeren und hochmolekularen organischen Materialien.

Zur Herstellung nicht starrer Formlinge oder zur Verringerung ihrer Sprödigkeit kann man den hochmolekularen Substanzen vor der Verformung sogenannte Weichmacher zusetzen. Weichmacher können beispielsweise sein: Ester der Phosphorsäure, Phthalsäure und Sebazinsäure. Die Weichmacher können vor, während oder nach dem Einfärben der hochmolekularen Substanzen mit den erfindungsgemäßen DPP-Polymeren zugesetzt werden.

Zur Erzielung verschiedener Farbtöne kann man die erfindungsgemäßen DPP-Polymere vorteilhaft in Abmischung mit Füllstoffen, transparenten und deckenden Weiß-, Bunt-und/oder Schwarzpigmenten sowie auch herkömmlichen Glanzpigmenten in der gewünschten Menge einsetzen.

Zur Herstellung von Lacken, Anstrichstoffen, Tinten und Druckfarben dispergiert oder löst man im allgemeinen die entsprechenden hochmolekularen organischen Substanzen wie Bindemittel, Kunstharzdispersionen etc. und die erfindungsgemäßen DPP-Polymere, gewünschtenfalls zusammen mit üblichen Zusatzstoffen wie Füllmitteln, Lackhilfsmitteln, Siccativen, Weichmachern und/oder zusätzlichen Pigmenten, in einem gemeinsamen Lösungsmittel oder Lösungsmittelgemisch. Man kann dabei so verfahren, daß man die einzelnen Komponenten für sich oder auch mehrere gemeinsam dispergiert oder löst, und erst hierauf alle Komponenten zusammenbringt, oder alles auf einmal zugibt.

Bei der Applikation im Druck kann man alle industrieüblichen Druckverfahren wie Siebdruck, Tiefdruck, Bronzierdruck, Flexodruck und Offestdruck einsetzen.

Die folgenden Beispiele erläutern die Erfindung.

### Herstellung von DPP-Monomeren

Beispiel 1a: Zu 290 ml tert.-Amylalkohol werden unter Stickstoffbegasung 16,6 g Natrium und 0,24 g Sulfobernsteinsäure-bis-2-acetyl-hexylester-Natriumsalz zugegeben. Das Gemisch wird unter leichtem Rühren bei 95-102°C erwärmt. Sobald das Natrium geschmolzen ist, wird die Emulsion während 3 bis 5 Stunden bei 95-102°C kräftig gerührt. Die so erhaltene Lösung versetzt man einerseits mit 64,0 g (0,48 Mol) p-Methoxybenzonitril. Mittels einer Dosierpumpe gibt man bei 105-110°C während 3 Stunden 484,8 g (2,4 Mol) in 24 ml tert.-Amylalkohol gelösten Bemsteinsäure-diisopropylester. Das entstehende Isopropanol wird laufend abdestilliert. Nach beendeter Zudosierung wird das Gemisch noch 2 Stunden bei 105-110°C gehalten, auf 65°C abgekühlt, mit Methanol verdünnt, langsam mit Eisessig neutralisiert und kurz auf Rückflusstemperatur erhitzt. Die erhaltene Pigment-Suspension wird bei ca. 50°C filtriert. Schliesslich wäscht man den Rückstand farblos mit Methanol und Wasser und trocknet ihn bei 80°C im Vakuum. Man erhält mit guter Ausbeute das gewünschte Produkt der Formel

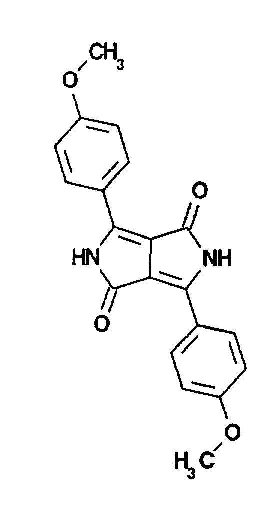

Das NMR-Spectrum in CDCl₃/p.a. stimmt mit der Zielstruktur voll überein.

Beispiel 1b: 20,21g (0,060 Mol) des Produktes von Beispiel 1a werden in einem gut mit Stickstoff gespülten Sulfierkolben direkt eingewogen, dann werden 270 ml Dimethylformamid dazugegeben und unter Rühren und Stickstoffzufuhr auf 130-135°C erhitzt. Nach 30 Minuten werden 12,44 g (0,090 Mol) Kaliumcarbonat (bei 250°C getrocknet) zugegeben. In die dunkelviolette Suspension werden 15,18 g (0,090 Mol) in 30 ml Dimethylformamid gelöstes 1-(2-Bromo-ethoxy)-2-ethoxy-ethan innert 15 Minuten zugetropft. Die entstandene dunkelbraune Suspension wird 4½ Stunden bei 130-135°C gerührt, dann auf Raumtemperatur abgekühlt und über Nacht weitergerührt. Danach wird filtriert und der Filterrück-stand wird mit Dimethylformamid gewaschen. Das Filtrat wird im Rotationsverdampfer bis auf etwa 150 ml eingeengt. Das Produkt kristallisiert aus und wird aus 200 ml Ethanol umkristallisiert und bei 40-50°C im Vakuumtrockenschrank getrocknet. Man erhält 8,42 g (30% d.Th.) des Produktes der Formel

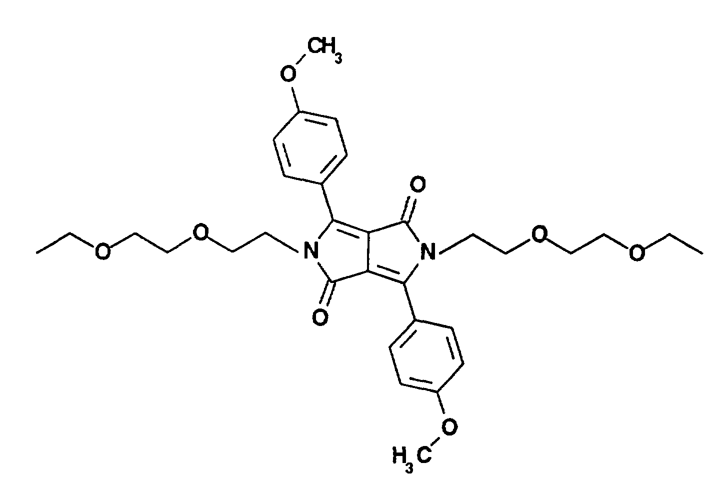

Das NMR-Spectrum in CDCl₃/p.a. stimmt mit der Zielstruktur voll überein.

Beispiel 1c: Bor-Tribromid (36 ml, 0,060 Mol) in CH₂Cl₂ (50 ml) werden langsam zu einer Lösung des Produktes von Beispiel 1b (5 g, 0,010 Mol) in CH₂Cl₂ (50 ml) bei -78°C beigegeben. Die Lösung wird langsam auf Raumtemperatur aufgewärmt und während 24 Stunden gerührt. Wasser (50 ml) wird langsam zur Lösung beigegeben. Die resultierende Mischung wird mit Dichlormethan extrahiert (3x60 ml). Die kombinierte organische Phase wird über Magnesiumsulfat getrocknet und konzentriert. Das Rohprodukt wird aus Dimethylsulfoxyd umkristallisiert. Man erhält 3,9 g des Produktes der Formel

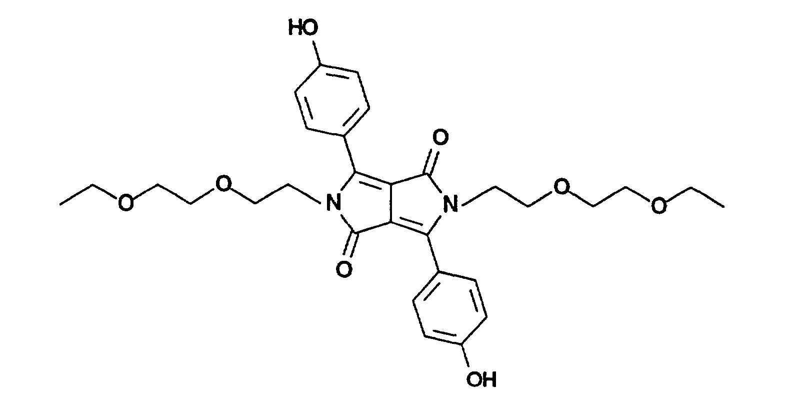

Das NMR-Spectrum in CDCl₃/p.a. stimmt mit der Zielstruktur voll überein.

Beispiel 2: 19,55 g (0,04 Mol) des Produktes von Beispiel 1c, 0,04 g (2x10⁻⁴ Mol) Phenothiazin als Katalysator und 380 ml Dichlorethan werden unter Stickstoff in einem Sulfierkolben vorgelegt und zum Rückfluss geheizt. Der trüben Lösung werden innert etwa 30 Minuten unter Rückfluss (80°C) und Rühren 14,48 g (0,16 Mol) Acrylsäurechlorid zugetropft. Nach beendeter Zugabe wird mit 20 ml Dichlorethan nachgespült. Die Lösung wird 7 Stunden am Rückfluss gerührt, anschliessend auf Raumtemperatur und über Nacht stehen gelassen. Die orangerote, ganz leicht trübe Lösung wird im Scheidetrichter 5 Mal mit 100 ml 5%iger Natronlauge und 2 Mal mit deionisiertem Wasser gewaschen und mit MgSO₄•H₂O getrocknet und filtriert. Das Filtrat wird im Rotationsverdampfer vollständig eingeengt. Das zurückgebliebene rote Öl erstarrt über Nacht zu einer festen Masse, die aus Ethanol umkristallisiert wird. Man erhält 23,1 g (96,7% d.Th.) eines kristallinen Produktes der Formel

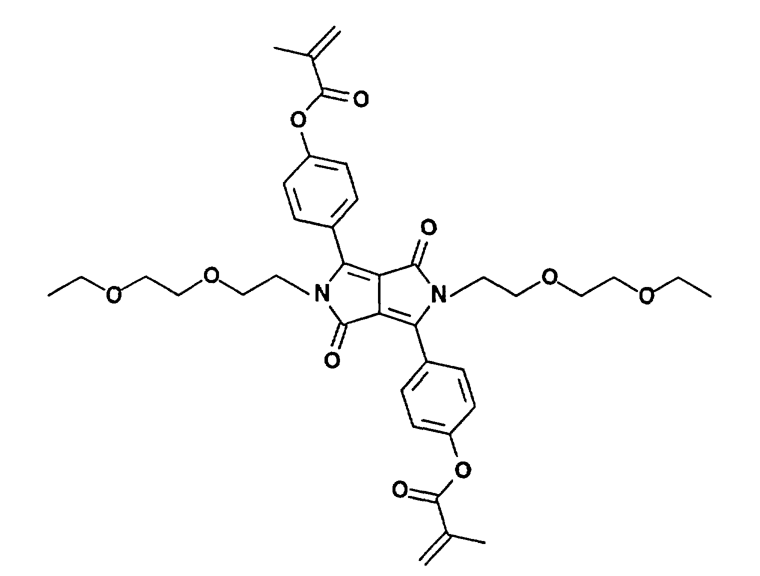

Das NMR-Spectrum in CDCl₃/p.a. stimmt mit der Zielstruktur voll überein

Beispiel 3a : In einem Sulfierkolben mit Rührer und Dean-Stark-Abscheider mit Kondensator werden 53 g (0,40 Mol) 4-Cyanobenzaldehyd, 34,5 ml (0,48 Mol) 1,3-Propandiol, 9,19 g (0,048 Mol) Tosyl-Säure und 1 I Benzol vorgelegt. Die Mischung wird unter Rückfluss während 5 Stunden gerührt. Während dieser Zeit werden ca. 7 ml Wasser im Abscheider abgeschieden. Das resultierende Reaktionsgemisch wird auf Raumtemperatur abgekühlt und extrahiert, einmal mit einer 2%igen Natrium-Hydrogen-Carbonat-Lösung (2x 500 ml) und dann mit H₂O dest. (2x 500 ml). Die organische Phase wird über MgSO₄ getrocknet. Das Lösungsmittel wird unter Vakuum entfernt und das feste Produkt unter Vakuum bei 60°C getrocknet. Man erhält 68,2 g (90,1% d.Th.) einer weissen Verbindung der Formel 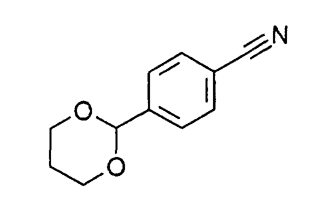 mit einem Schmelzpunkt von 108 - 109°C.

| Analyse | C | H | N |
|---|---|---|---|
| berechnet | 69,83% | 5,86% | 7,40% |
| gefunden | 69,88% | 5,87% | 7,10% |

Beispiel 3b: Eine Mischung aus 6,90 g (0,3 Mol) Natrium und 600 ml tert.-Amylalkohol wird unter Stickstoff gerührt und auf 100°C aufgeheizt. Bei dieser Temperatur ist das Natrium geschmolzen. Nach weiterem Rühren während 16 Stunden wird eine klare Lösung erhalten. 37,8 g (0,2 Mol) des Produktes von Beispiel 3a werden dazugegeben und anschliessend 20,2 g (0,1 Mol) Bemsteinsäurediisopropylester. Die Reaktion läuft während weiteren 28 Stunden bei derselben Temperatur weiter. Anschliessend wird das Reaktionsgemisch auf Raumtemperatur abgekühlt und in eine Mischung bestehend aus 45 ml Essigsäure und 1400 ml Methanol eingegeossen. Das Produkt (feine Kristalle) wird durch Filtration isoliert, zuerst mit Methanol und dann mit Wasser gewaschen und unter Vakuum bei 80°C getrocknet. Man erhält 18,0 g (39% d.Th.) eines dunkelroten Produktes der Formel

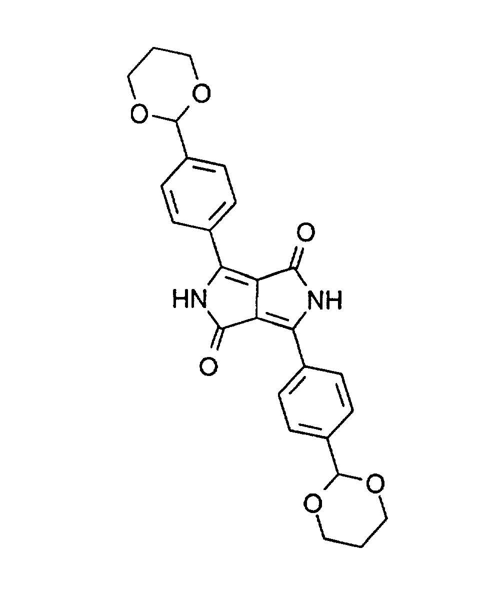

| Analyse | C | H | N |
|---|---|---|---|
| berechnet | 67,82% | 5,25% | 6,08% |
| gefunden | 67,71% | 5,29% | 6,07% |

Beispiel 3c: Eine Suspension bestehend aus 6,70 g (0,0145 Mol) des Produktes von Beispiel 3b, 300 ml (0,6 Mol) einer wässrigen 2M-Salzsäure-Lösung und 350 ml Tetrahydrofuran wird während 70 Stunden unter Rückfluss gerührt und danach auf Raumtemperatur abgekühlt. Das dunkelrote Produkt wird durch Filtration isoliert, mit Methanol und Wasser gewaschen und unter Vakuum bei 60°C getrocknet. Man erhält 4,86 g (97% d.Th.) eines Produktes der Formel

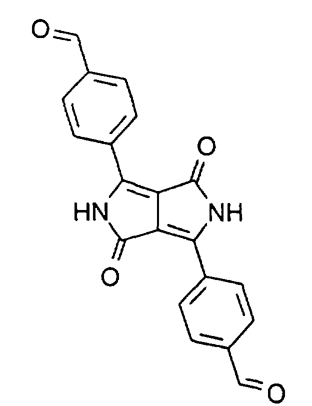

| Analyse | C | H | N |
|---|---|---|---|
| berechnet | 69,76% | 3,51% | 8,14% |
| gefunden | 68,58% | 3,66% | 8,09% |

Beispiel 4: Eine Reaktionsmischung bestehend aus 4,14 g (0,012 Mol) des Produktes von Beispiel 3c, 0,85 g (0,0068 Mol) 4-Dimethylaminopyridin und 13,8 g (0,063 Mol) Di-tert-butyl-dicarbonat in 250 ml Tetrahydrofuran wird während 24 Stunden bei Raumtemperatur gerrührt. Weitere 5,2 g (0,024 Mol) Di-tert-butyl-dicarbonat werden dazugegeben und die Reaktionsmischung während weiteren 5 Stunden weitergerührt. Das Lösungsmittel wird bei reduziertem Druck verdampft. Der feuchte Rückstand wird mit 25 ml Methanol vermischt, während 30 Minuten gerührt, filtriert, mit mehr Methanol gespült und schliesslich unter Vakuum bei Raumtemperatur getrocknet. Man erhält mit guter Ausbeute ein orangefarbiges kristallines Produkt der Formel

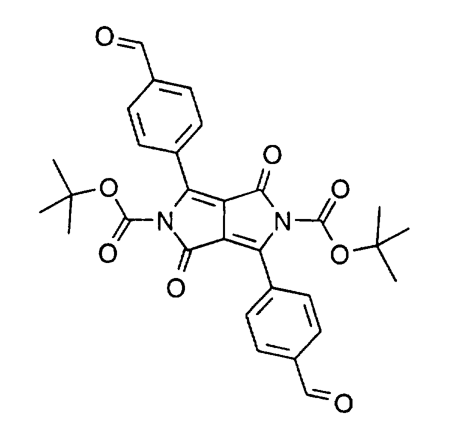

### Herstellung von DPP-Polymeren

Beispiel 5 : 5,13 g (0,01 Mol) des Produktes von Beispiel 1c und 60 ml Chlorbenzol werden unter Stickstoff in einem Sulfierkolben vorgelegt und zum leichten Rückfluss aufgeheizt. Bei 129°C werden weitere 30 ml Chlorbenzol dazugegeben. In die leicht trübe Lösung werden bei 130°C 1,68 g (0,01 Mol) Hexamethylendiisocyanat innerhalb von 15 Minuten zugetropft. 40 Minuten nach beendeter Zugabe (und bei unveränderter Temperatur) liegt eine praktisch klare Lösung vor. Nach 75 Minuten Reaktionszeit werden 0,013 g Dimethylcyclohexylamin (1,0 mol-%ige Lösung in Chlorbenzol) als Katalysator zugegeben. Nach weiteren 3¼ Stunden ist die Lösung in eine feine Suspension übergegangen. Es wird über Nacht bei der gleichen Temperatur weitergerührt und anschliessend auf Raumtemperatur abgekühlt. Die orangerot gefärbte Suspension wird filtriert und das Produkt bei 60-70°C im Vakuum getrocknet. Man erhält 6,3 g (92,5% d.Th.) des gewünschten Polyurethans.

Im IR (KBr Pressling) kann die charakteristische Urethan-Bande bei 2330 cm⁻¹ klar erkannt werden.

### Beispiel 6: Photohärtung des DPP-Bisacrylat-Monomers

0,80 g des Produktes von Beispiel 2 werden mit 7,2 g Cibatool®SL 5154* (eine Mischung, enthaltend Acrylat-Monomere, CIBA-GEIGY AG) bei ca. 60°C vermischt und die daraus resultierende orange-rote Lösung im Vakuum kurz entgast.

### B) Applikation

Mit einem Erichsen Ziehdreieck werden ca.100 um dicke Filme auf Glas gezogen, die anschliessend mit Hilfe einer Hoenle-UV-Lampe aus einer Distanz von 20 cm und Einstellung auf 60% belichtet werden.

Nach einer Belichtungszeit von 30 Minuten ist das DPP-Bisacrylat in dieser Formulierung in Dimethylformamid vollkommen unlöslich, was auf eine vollständige Vernetzung hinweist.

## Patentansprüche

1. 1,4-Diketopyrrolopyrrole der Formel 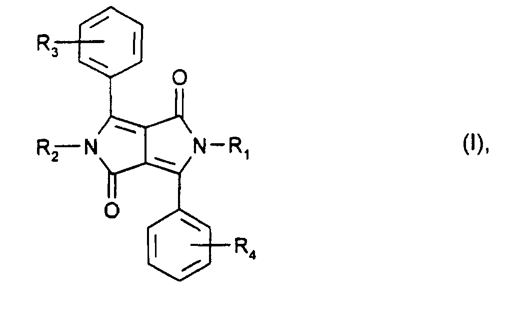 worin R₁ und R₂ unabhängig voneinander Wasserstoff, C₁₂-C₂₄-Alkyl, ein- oder mehrfach durch O oder S unterbrochenes C₆-C₂₄-Alkyl oder eine Gruppe der Formel 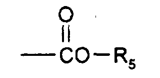 bedeuten, worin R₅ C₄-C₁₈-Alkyl oder C₅-C₁₀-Cycloalkyl ist, R₃ OH, SH, NH₂, CHO, NCO, Hydroxyphenyl, -CH=CH₂, -CH=CH-COOR₆, 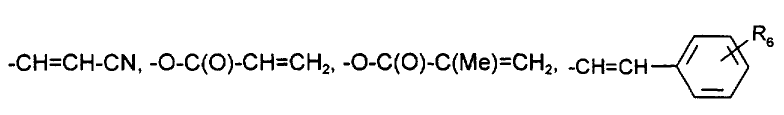 , oder einen Rest der Formeln
-A-(CH₂)ₘ-CH=CH-(CH₂)ₙ-CH₃ (II),
oder
-A-(Y)ₚ-X-(Z)ᵣ-Q (III)
bedeutet, worin A -O- oder -NH- ist, m und n unabhängig voneinander eine ganze Zahl zwischen Null und 12 und p und r unabhängig voneinander Null oder 1 bedeuten,
X Methylen, ununterbrochenes oder ein- oder mehrfach durch -O- und/oder -S-, -NH-, Phenylen, -COO-, -CONH-, 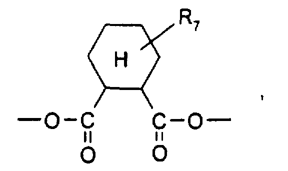 worin R₇ Wasserstoff oder Methyl ist, unterbrochenes C₂-C₁₈-Alkylen ist, 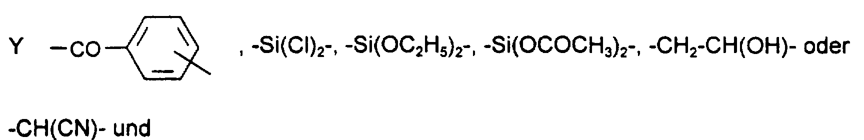 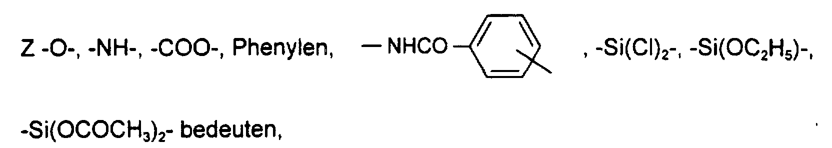 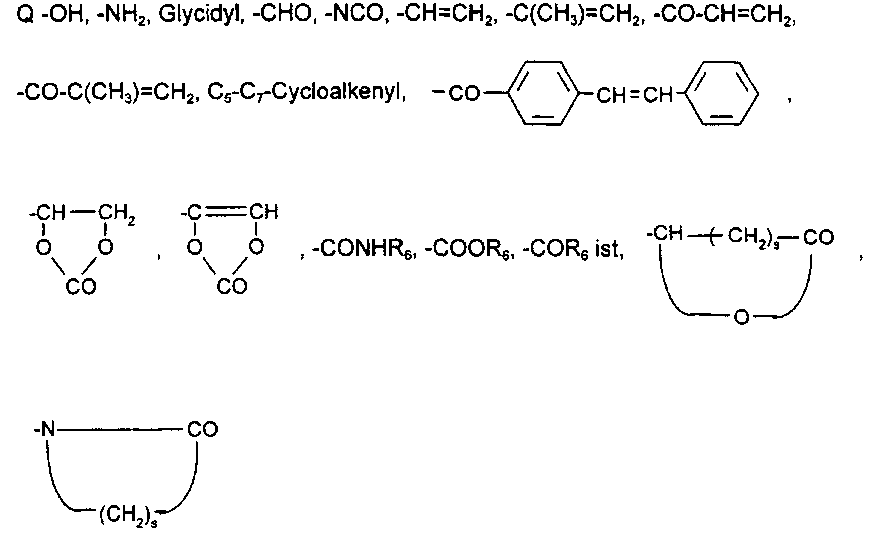 wobei s eine ganze Zahl von 1 bis 6 und R₆ Wasserstoff oder C₁-C₆Alkyl ist, bedeutet,
R₄, wenn R₁ und R₂ Wasserstoff bedeuten, ununterbrochenes oder ein- oder mehrfach durch O oder S unterbrochenes, direkt oder über O oder S am Benzolring gebundenes C₆-C₂₄-Alkyl ist und
wenn R₁ und/oder R₂ C₁₂-C₂₄-Alkyl, ein- oder mehrfach durch O oder S unterbrochenes C₆-C₂₄-Alkyl oder eine Gruppe 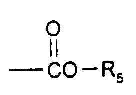 sind, Wasserstoff, Halogen, Methyl, Methoxy, CN, Phenyl oder gleich wie R₃ ist.

2. Diketopyrrolopyrrole gemäss Anspruch 1, der Formel l, dadurch gekennzeichnet, daß R₁ und R₂ Wasserstoff sind und R₄ direkt oder über O am Benzolring gebundenes C₆-C₁₈-Alkyl oder eine Gruppe -C(CH₂CH₂C)ₓCH₂CH₃ bedeutet, worin x 1, 2 oder 3 ist.

3. Diketopyrrolopyrrole gemäss Anspruch 1, der Formel l, dadurch gekennzeichnet, daß mindestens einer der Reste R₁ und R₂ C₁₂-C₁₈-Alkyl, eine Gruppe (CH₂CH₂O)ₓCH₂CH₃, worin x 1, 2 oder 3 ist, oder eine Gruppe der Formel 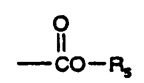 bedeutet, wobei R₅ die in Anspruch 1 gegebent Bedentung hat, und
R₄ Wasserstoff oder die gleiche Bedeutung wie R₃ hat.

4. Diketopyrrolopyrrole gemäss Anspruch 1, der Formel l, dadurch gekennzeichnet, daß R₃ OH, NH₂ oder einen Rest der Formel
-X-(O)ᵣ-Q (IV)
bedeutet, worin
x ununterbrochenes oder 1, 2 oder 3 Mal durch O und/oder einmal durch 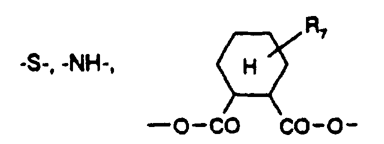
unterbrochenes C₄-C₁₂-Alkylen ist,
r und R₇ die in Anspruch 1 angegebene Bedeutung haben, und
Q -OH; -CH=CH₂, -C(CH₃)=CH₂, -CO-CH=CH₂ oder -CO-C(CH₃)=CH₂ bedeutet.

5. Diketopyrrolopyrrole der Formel 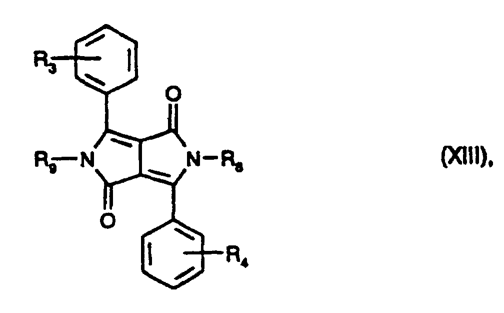 worin R₈ eine Gruppe
-(CH₂)ₘ-CH=CH-(CH₂)ₙ-CH₃
oder
-(Y)ₚ-X-(Z)ᵣ-Q
bedeutet und R₉ Wasserstoff oder R₈ ist, und R₃ und R₄ und X, Y, Z, Q, m, n, p und r die gleiche Bedeutung wie in Anspruch 1 haben.

6. Verfahren zur Herstellung von Diketopyrrolopyrrolen gemäß den Ansprüchen 1 bis 5 durch Umsetzung eines Bemsteinsäurediesters mit einem Nitril, dadurch gekennzeichnet, daß man (Schritt a) einen unsymmetrischen oder symmetrischen Bernsteinsäure-dialkyl-oder -diaryl-ester, oder einen Bernsteinsäure-monoalkyl-monoaryl-ester oder Bernsteinsäuredicyclohexylester mit einem Nitril der Formel 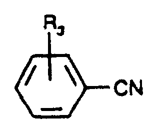 oder mit einer Mischung, bevorzugt einer äquimolaren Mischung, der Nitrile der Formeln 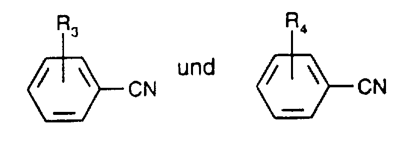 wobei R₃ und R₄ die gleiche Bedeutung wie in Anspruch 1 haben sowie zusätzlich übliche Schutzgruppen bedeuten können, im gewünschten Molverhältnis in einem organischen Lösungsmittel in Gegenwart einer starken Base umsetzt, und anschließend das Reaktionsprodukt hydrolysiert und gewünschtenfalls das gewünschte Produkt isoliert, sowie (Schritt b) gewünschtenfalls die erhaltenen Diketopyrrolopyrrole der Formel l, in denen R₁ und R₂ für Wasserstoff stehen, mit (b1) einem Dicarbonat der Formel XI, 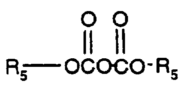 im gewünschten Molverhältnis umsetzt, oder (b2) im gewünschten Molverhältnis mit einer Halogenverbindung der Formel Xll, R₁-Hal, worin R₁ C₁₂-C₂₄-Alkyl, ein- oder mehrfach durch O oder S unterbrochenes C₆-C₂₄-Alkyl ist, oder (b3) im gewünschten Molverhältnis mit einer Halogenverbindung Hal-(CH₂)ₘ-CH=CH-(CH₂)ₙ-CH₃ oder Hal-(Y)ₚ-X-(Z)ᵣ-Q umsetzt, worin m, n, p, r, Y, X, Q und Z die gleiche Bedeutung wie in Anspruch 1 haben, und (Schritt c) gewünschtenfalls direkt vor oder nach Schritt b Diketopyrrolopyrrole der Formel l, in denen R₃ und/oder R₄ Schutzgruppen sind, in üblicher Weise zum entsprechenden gewünschten Zielmolekül umsetzt.

7. Verfahren zur Herstellung oder Modifizierung von Polymeren durch Polyreaktion oder polymeranaloge Reaktion, dadurch gekennzeichnet, daß man ein Diketopyrrolopyrrol der Formel l, gewünschtenfalls in Gegenwart eines Comonomeren oder eines polyreaktionsfähige Gruppen tragenden Polymeren, polymerisiert.

8. Verwendung von Diketopyrrolopyrrolen gemäss den Ansprüchen 1 bis 5 oder hergestellt gemäss Anspruch 6 zur Herstellung von Polymeren durch eine Polyreaktion oder eine polymeranaloge Reaktion.

9. Mit Diketopyrrolopyrrolen gemäss den Ansprüchen 1 bis 5 oder hergestellt gemäss Anspruch 6 hergestellte oder modifizierte Polymere.

## Claims

1. A 1,4-diketopyrrolopyrrole of the formula 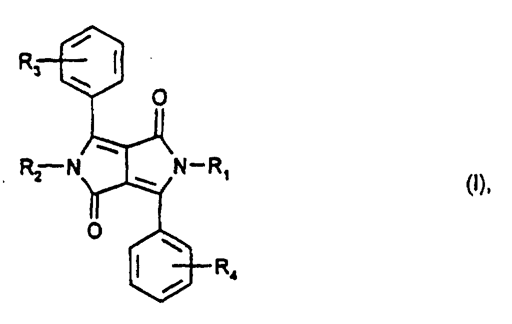 in which R₁ and R₂ independently of one another are hydrogen, C₁₂-C₂₄alkyl, C₆-C₂₄alkyl which is interrupted one or more times by O or S, or are a group of the formula 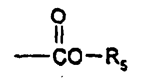 in which R₅ is C₄-C₁₈alkyl or C₅-C₁₀cycloalkyl,
R₃ is OH, SH, NH₂, CHO, NCO, hydroxyphenyl, -CH=CH₂, -CH=CH-COOR₆, -CH=CH-CN, 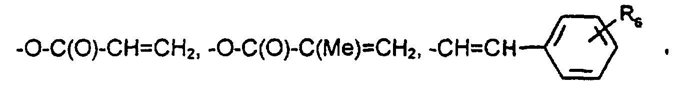 or is a radical of the formula
-A-(CH₂)ₘ-CH=CH-(CH₂)ₙ-CH₃ (II)
or
-A-(Y)ₚ-X-(Z)ᵣ-Q (III),
in which A is -O- or -NH-, m and n independently of one another are an integer from zero to 12, and p and r independently of one another are zero or 1,
X is methylene or is C₂-C₁₈alkylene which is uninterrupted or is interrupted one or more times by -O- and/or -S-, -NH-, phenylene, -COO-, -CONH- or by 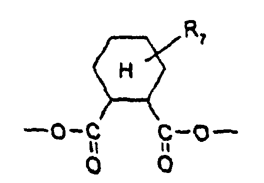 in which R₇ is hydrogen or methyl, 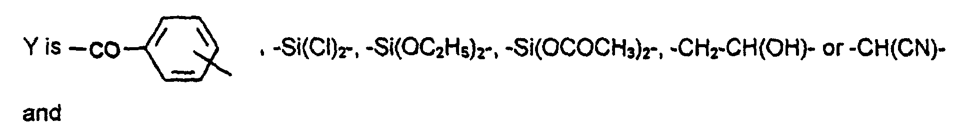 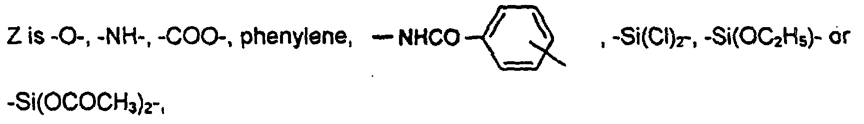 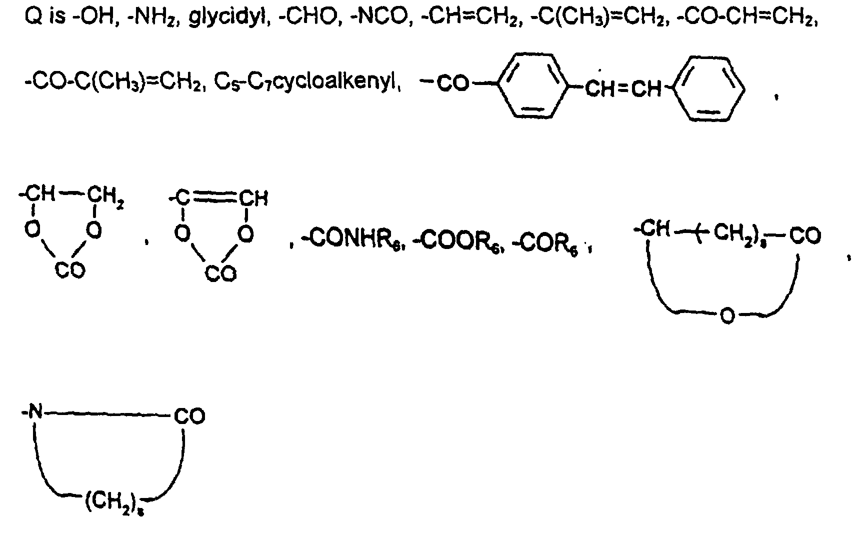 where s is an integer from 1 to 6 and R₆ is hydrogen or C₁-C₆alkyl,
R₄, if R₁ and R₂ are hydrogen, is C₆-C₂₄alkyl which is uninterrupted or is interrupted one or more times by O or S and which is bonded to the benzene ring directly or by way of O or S, and,
if R₁ and/or R₂ are C₁₂-C₂₄alkyl, C₆-C₂₄alkyl which is interrupted one or more times by O or S or are a group 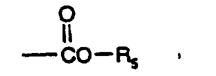 R₄ is hydrogen, halogen, methyl, methoxy, CN or phenyl or is the same as R₃.

2. A diketopyrrolopyrrole according to claim 1 of formula I, wherein R₁ and R₂ are hydrogen and R₄ is C₆-C₁₈alkyl bonded to the benzene ring directly or by way of O or is a group -O(CH₂CH₂O)ₓCH₂CH₃ in which x is 1, 2 or 3.

3. A diketopyrrolopyrrole according to claim 1 of formula I, wherein at least one of the radicals R₁ and R₂ is C₁₂-C₁₈alkyl, a group (CH₂CH₂O)ₓCH₂CH₃ in which x is 1, 2 or 3, or a group of the formula 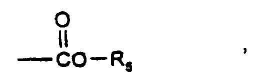 where R₅ is as defined in claim 1, and R₄ is hydrogen or is as defined for R₃.

4. A diketopyrrolopyrrole according to claim 1 of formula I, wherein R₃ is OH, NH₂ or a radical of the formula
-X-(O)ᵣ-Q (IV),
in which
X is C₄-C₁₂alkylene which is uninterrupted or is interrupted 1, 2 or 3 times by O and/or once by 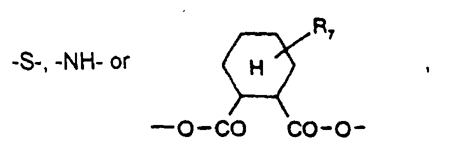
r and R₇ are as defined in claim 1, and
Q is -OH, -CH=CH₂, -C(CH₃)=CH₂, -CO-CH=CH₂ or -CO-C(CH₃)=CH₂.

5. A diketopyrrolopyrrole of the formula 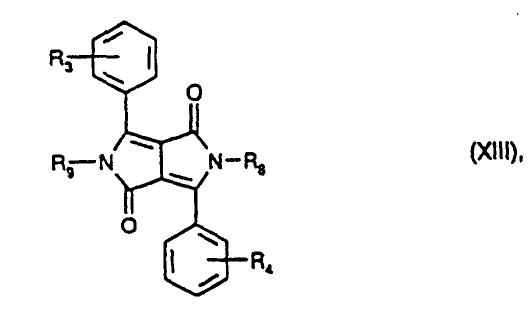 in which R₈ is a group
-(CH₂)ₘ-CH=CH-(CH₂)ₙ-CH₃
or
-(Y)ₚ-X-(Z)ᵣ-Q
and R₉ is hydrogen or is R₈, and R₃ and R₄ and X, Y, Z, Q, m, n, p and r are as defined in claim 1.

6. A process for the preparation of a diketopyrrolopyrrole according to claims 1 to 5 by reaction of a succinic diester with a nitrile, which comprises (step a) reacting an asymmetric or symmetric dialkyl or diaryl succinate, or a monoalkyl monoaryl succinate or dicyclohexyl succinate with a nitrile of the formula 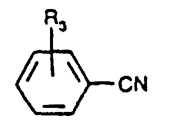 or with a mixture, preferably an eauimolar mixture. of the nitriles of the formulae 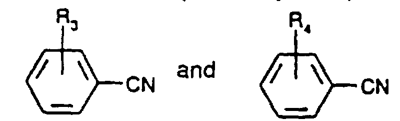 where R₃ and R₄ are as defined in claim 1 and may, in addition, be customary protective groups, in the desired molar ratio in an organic solvent in the presence of a strong base, and then hydrolysing the reaction product and, if desired, isolating the desired product, and (step b) if desired reacting the resulting diketopyrrolopyrrole of formula I in which R₁ and R₂ are hydrogen with (b1) a dicarbonate of formula XI, 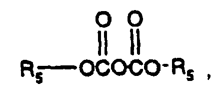 in the desired molar ratio, or (b2) in the desired molar ratio with a halogen compound of formula XII, R₁-Hal, in which R₁ is C₁₂-C₂₄alkyl, or C₆-C₂₄alkyl which is interrupted one or more times by O or S, or (b3) in the desired molar ratio with a halogen compound Hal-(CH₂)ₘ-CH-CH-(CH₂)ₙ-CH₃ or Hal-(Y)ₚ-X-(Z)ᵣ-Q, in which m, n, p, r, Y, X, Q and Z are as defined in claim 1, and (step c) if desired, immediately prior to or following step b, reacting a diketopyrrolopyrrole of formula I in which R₃ and/or R₄ are protective groups in customary manner to give the corresponding desired target molecule.

7. A process for the preparation or modification of polymers by a polyreaction or a polymer-analogous reaction, which comprises polymerizing a diketopyrrolopyrrole of formula I, if desired in the presence of a comonomer or of a polymer that carries groups capable of polyreaction.

8. The use of a diketopyrrolopyrrole according to claims 1 to 5 or prepared according to claim 6 for the preparation of polymers by a polyreaction or a polymer-analogous reaction.

9. A polymer prepared or modified using a diketopyrrolopyrrole according to claims 1 to 5 or a diketopyrrolopyrrole prepared according to claim 6.

## Revendications

1. 1,4-Dicétopyrrolopyrroles de formule 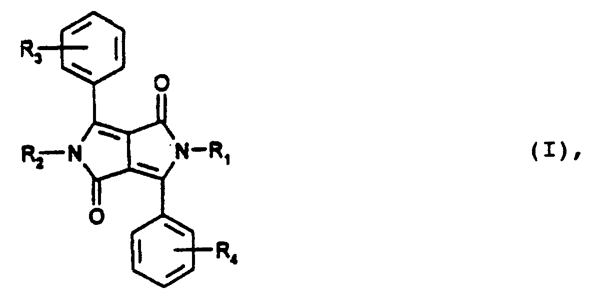 où R₁ et R₂ représentent, indépendamment l'un de l'autre, un atome d'hydrogène, des groupes alkyle en C₁₂-C₂₄, alkyle en C₆-C₂₄ interrompu une ou plusieurs fois par des atomes de O ou S ou un groupe de formule 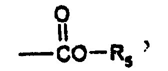 où R₅ représente un groupe alkyle en C₄-C₁₈ ou cycloalkyle en C₅-C₁₀,
R₃ représente des groupes OH, SH, NH₂, CHO, NCO, hydroxyphényle, -CH=CH₂, -CH=CH-COOR₆, -CH=CH-CN, 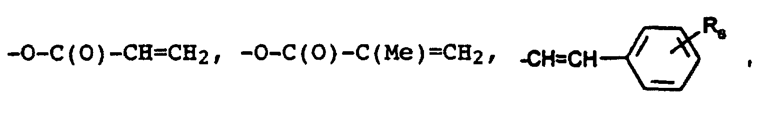 ou un reste de formules
-A-(CH₂)ₘ-CH=CH-(CH₂)ₙ-CH₃ (II),
ou
-A-(Y)ₚ-X-(Z)ᵣ-Q (III),
où A représente des groupes -O- ou -NH, m et n valent indépendamment l'un de l'autre un entier compris entre zéro et 12 et p et r valent, indépendamment l'un de l'autre, zéro ou 1,
X représente un groupe méthylène, un groupe phénylène non interrompu ou interrompu une ou plusieurs fois par des groupes -O- et/ou -S-, -NH, représente des groupes -COO-, -CONH-, 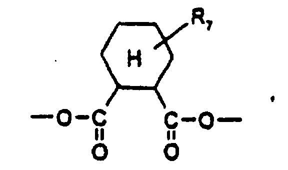 où R₇ représente un atome d'hydrogène ou un groupe méthyle, un groupe alkylène en C₂-C₁₈ interrompu, 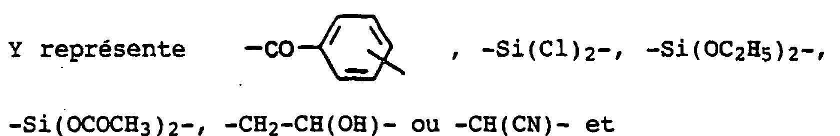 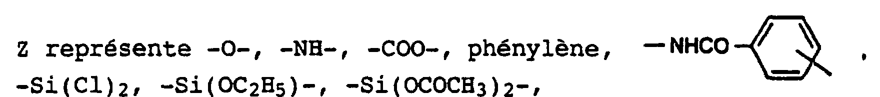 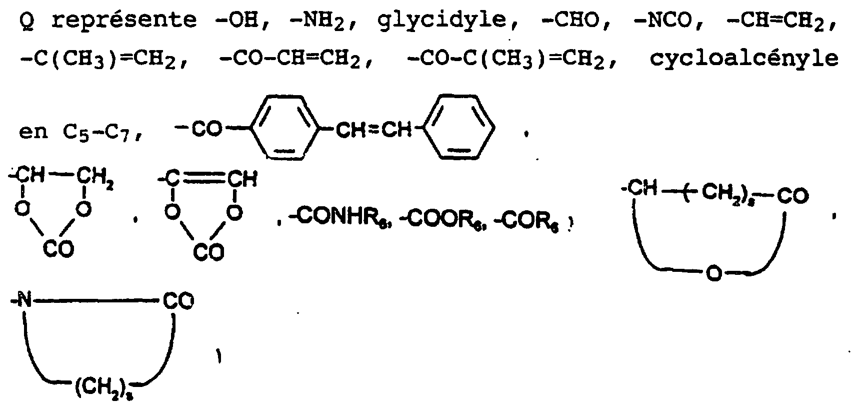 s étant un entier de 1 à 6 et R₆ représente un atome d'hydrogène ou un groupe alkyle en C₁-C₆,
R₄ représente, lorsque R₁ et R₂ représentent un atome d'hydrogène, un groupe alkyle en C₆-C₂₄ non interrompu ou interrompu une ou plusieurs fois par O ou S, lié directement ou par l'intermédiaire des atomes de O ou S au cycle benzène et
lorsque R₁ et/ou R₂ représentent un groupe alkyle en C₁₂-C₂₄, un groupe alkyle en C₆-C₂₄ interrompu une ou plusieurs fois par O ou S ou un groupe 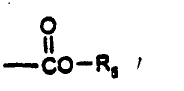 un atome d'hydrogène, un atome d'halogène, des groupes méthyle, méthoxy, CN, phényle ou est identique à R₃.

2. Dicétopyrrolopyrroles selon la revendication 1, de formule I, caractérisés en ce que R₁ et R₂ représentent un atome d'hydrogène et R₄ représente un groupe alkyle en C₆-C₁₈ lié directement ou par l'intermédiaire de l'atome d'oxygène au cycle benzène ou représente un groupe -O(CH₂CH₂O)ₓCH₂CH₃, où x vaut 1, 2 ou 3.

3. Dicétopyrrolopyrroles selon la revendication 1, de formule I, caractérisés en ce que au moins l'un des restes R₁ et R₂ représente un groupe alkyle en C₁₂-C₁₈, un groupe (CH₂CH₂O)ₓCH₂CH₃ où x vaut 1, 2 ou 3, ou un groupe de formule 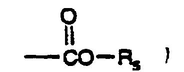 où R₅ possède la signification donnée à la revendication 1, R₄ représente un atome d'hydrogène ou possède la même signification que R₃.

4. Dicétopyrrolopyrroles selon la revendication 1, de formule I, caractérisés en ce que R₃ représente des groupes OH, NH₂ ou un reste de formule
-X-(O)ᵣ-Q (IV)
où
X représente un groupe alkylène en C₄-C₁₂ non interrompu ou interrompu 1, 2 ou 3 fois par des groupes O et/ou une fois par 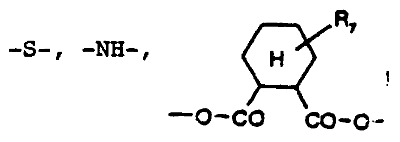
r et R₇ possèdent la signification donnée à la revendication 1, et
Q représente -OH ; -CH=CH₂ ; -C(CH₃)=CH₂ ; -CO-CH=CH₂ ou -CO-C(CH₃)=CH₂.

5. Dicétopyrrolopyrroles de formule 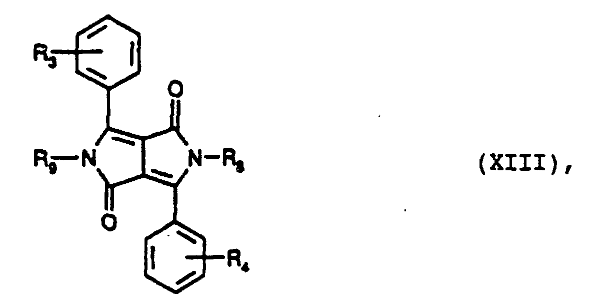 où R₈ représente un groupe
- (CH₂)ₘ-CH=CH-(CH₂)ₙ-CH₃
ou
-(Y)ₚ-X-(Z)ᵣ-Q,
et R₉ représente un atome d'hydrogène ou R₈, et R₃, R₄, X, Y, Z, Q, m, n, p et r possèdent la même signification que celle donnée à la revendication 1.

6. Procédé pour la préparation de dicétopyrrolopyrroles selon les revendications 1 à 5, par réaction d'un diester de l'acide succinique sur un nitrile, caractérisé en ce que (stade a) on fait réagir un succinate de dialkyle ou de diaryle asymétrique ou symétrique, ou un succinate de monoalkyl-monoaryle ou succinate de dicyclohexyle sur un nitrile de formule 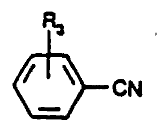 ou sur un mélange, de préférence un mélange équimolaire, des nitriles de formules 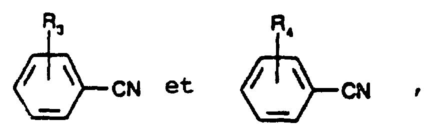 où R₃ et R₄ possèdent la même signification que celle donnée à la revendication 1, ainsi que pouvant représenter de plus des groupes protecteurs usuels, en rapport molaire voulu, dans un solvant organique, en présence d'une base forte et ensuite, on hydrolyse le produit réactionnel et, si on le souhaite, on isole le produit voulu, ainsi que (stade b), si on le souhaite, on peut faire réagir les dicétopyrrolopyrrole de formule I, où R₁ et R₂ représentent un atome d'hydrogène, sur (b1) un dicarbonate de formule XI, 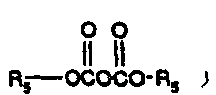 en rapport molaire voulu, ou (b2) en rapport molaire voulu sur un composé halogéné de formule XII, R₁-Hal, où R₁ représente un groupe alkyle en C₁₂-C₂₄, un groupe alkyle en C₆-C₂₄ interrompu une ou plusieurs fois par des atomes O ou S, ou (b3) on fait réagir en rapport molaire voulu sur un composé halogéné Hal-(CH₂)ₘ-CH=CH-(CH₂)ₙ-CH₃ ou Hal-(Y)ₚ-X-(Z)ᵣ-Q, où m, n, p, r, Y, X, Q et Z possèdent les mêmes significations que celles données à la revendication 1, et (stade c) si on le souhaite, on fait réagir directement avant ou après le stade b des dicétopyrrolopyrroles de formule I, où R₃ et/ou R₄ représentent des groupe protecteurs, de façon usuelle pour obtenir la molécule recherchée correspondante voulue.

7. Procédé pour la préparation ou la modification de polymères par polyréaction ou réaction analogue à la polymérisation, caractérisé en ce qu'on polymérise un dicétopyrrolopyrrole de formule I, si on le souhaite en présence d'un comonomère ou d'un polymère portant des groupes capables de polyréaction;

8. Utilisation de dicétopyrrolopyrroles selon les revendications 1 à 5 ou préparés selon la revendication 6 pour la préparation de polymères par une polyréaction ou une réaction analogue à la polymérisation.

9. Polymères préparés ou modifiés par des dicétopyrrolopyrroles selon les revendications 1 à 5 ou préparés selon la revendication 6.
